# EUROPEAN PATENT SPECIFICATION

(11) **EP 3 578 558 B1**
(45) Date of publication and mention of the grant of the patent: **24.02.2021**
(21) Application number: 19177830.7
(22) Date of filing: 03.06.2019
(51) Int. Cl.: C07D 413/14, A61Q 17/04, A61K 31/53

(54) **HIGHLY EFFECTIVE PHOTOPROTECTIVE AGENTS**
HOCH EFFEKTIVE PHOTOREAKTIVE VERBINDUNGEN
AGENTS PHOTORÉACTIVES TRÈS EFFICACES

(30) Priority: 05.06.2018 IT 201800006038
(43) Date of publication of application: 11.12.2019
(73) Proprietor: 3V SIGMA S.p.A., 20121 Milano (IT)
(72) Inventor: BERTE', Ferruccio, 24121 Bergamo (IT); SECCOMANDI, Carlo, 24121 Bergamo (IT); BEMPORAD, Luca, 24035 Curno (BG) (IT)
(74) Representative: Bianchetti Bracco Minoja S.r.l.

(56) References cited:
- EP-A1- 0 570 838
- WO-A1-2013/156272
- US-A- 5 744 127

## Description

The present invention relates to s-triazine derivatives, formulations containing them, and their use as photoprotectors and light stabilisers.

### Prior art

Ultraviolet solar radiation is known to have a harmful effect on skin tissue. By using particular compounds called sunscreens, which absorb the UV part of solar radiation, harmful effects and aging of the skin can be prevented, or at least slowed.

Numerous substances have been studied and tested as protective agents. A great deal of patent literature exists, relating to compounds belonging to various chemical classes which absorb in the ultraviolet region, particularly radiation between 290 and 320 nm, called UV-B, and radiation between 320 and 400 nm, called UV-A. Both types of radiation are harmful to the skin, damaging it in different ways and at different depths. UV-A radiation is more penetrating than UV-B radiation.

Comparatively few of said compounds have proved suitable for practical application. They include p-methoxycinnamic acid and p-dimethylaminobenzoic acid esters, benzotriazoles and hydroxybenzophenones.

A drawback shared by all these compounds is their low ability to absorb radiation between 290 and 400 nm, which means that relatively large quantities are required to obtain the optimum photoprotective effect.

An excellent UV absorber should have the following characteristics:
1) High specific extinction E¹₁ at wavelengths ranging from 290 to 400 nm, allowing the use of low doses, resulting in cost savings and minimal toxicological risk
2) Light stability
3) Heat stability
4) Oxidation stability
5) Stability to different pHs
6) Good solubility in the basic substances commonly used for dermatological formulations
7) Negligible toxicity
8) Colour and odour compatible with the intended applications
9) High molecular weight, which reduces the probability of absorption by the skin and increases toxicological safety
10) Compatibility with the various substances generally used in dermatological formulations.

The efficacy of UV absorbers is characterised by the parameter E¹₁ (or E11), which corresponds to the extinction value measured at the maximum absorption wavelength of a solution containing 1% of the product in question, measured with an optical path of 1 cm. Such extinction is called "specific extinction".

Numerous derivatives of symmetrical triazine are already known, which can be used in a wide variety of technical applications and sectors due to their properties of absorbing UV rays, in particular UV-A and UV-B rays. Examples of said triazines are disclosed in DE 3206398, US 4 617 390, US 4 724 137, US 5 233 040, US 5 252 323, US 5 332 568, IT 1255729, US 5 346 691, US 5 393 517, EP 832642, US 5 744 127, US 5 759 525, US 5 801 244, US 6 018 044, US 6 193 960, US 2002085981 and US 2005143577.

In particular, DE 3206398 discloses s-triazine derivatives obtained by reacting trichlorotriazine with p-amino-benzoic acid esters, which absorb intensely in the UV-B region. Unfortunately, the solubility of said compounds in the solvents conventionally used to formulate sun creams is very low, which makes their practical use problematic and very difficult, especially when the percentage of photoprotector in the composition must be increased to prepare formulations with a high sun protection factor.

IT 1255729 discloses s-triazine derivatives obtained by reacting trichlorotriazine with p-amino-benzoic acid esters or amides with high specific extinction in the UV-B zone and improved solubility in solvents.

US 5744127 discloses s-triazine derivatives obtained by reacting trichlorotriazine with p-aminophenyl-benzoxazole derivatives with high specific extinction in the UV-A zone.

The same patent application also describes compounds that effectively absorb both UV-A and UV-B radiation. In said cases, as the effect of absorption of UV-A radiation is obtained by introducing p-aminophenyl-benzoxazole groups, the additional effect of UV-B absorption derives from the introduction of p-amino-benzoic groups. However, the addition of this second type of substituents into the molecule definitely limits the solubility of the substances obtained in the solvents typically used in cosmetic formulations, greatly reducing the possibility of their final use. The problem of finding a substance with high specific extinction in the UV-A and UV-B zones which is highly soluble in solvents and extensively compatible with the ingredients used in cosmetic formulations therefore still remains to be solved.

Sun protection factor (SPF) is a measurement of the photoprotective power of a sunscreen or a cosmetic formulation containing one or more sunscreens. The sun protection factor is the ratio between the MED (Minimal Erythema Dose) determined on protected skin and the MED determined on unprotected skin. It is directly correlated with specific extinction, and therefore also with the amount of photoprotector present in the cosmetic preparation.

### Description of the invention

It has now been found that the triazine compounds of general formula (I): 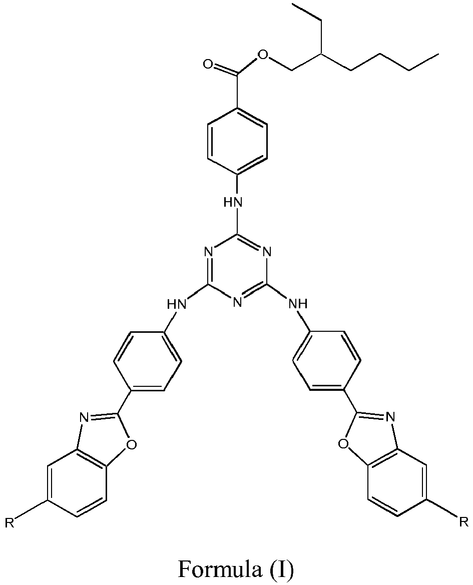 wherein the two R groups are independently branched C₅ alkyls, not only absorb very intensely in the UV-A zone and also cover part of the UV-B zone, but also exhibit improved solubility in the solvents most commonly used as ingredients of sunscreen formulations.

The invention also relates to the use of said compounds as sunscreens and photostabilisers, due to their ability to perform a surprising skin protection action against the harmful component of solar radiation, and formulations containing them.

The compounds of the invention, in addition to high absorption in the UV-A zone which also extends to the UV-B zone, and high solubility in the solvents most commonly used as ingredients of sunscreen formulations, also possess other advantageous characteristics, such as heat stability and non-toxicity associated with their high molecular weight.

### Detailed description of the invention

The invention relates to the triazine compounds of Formula (I): 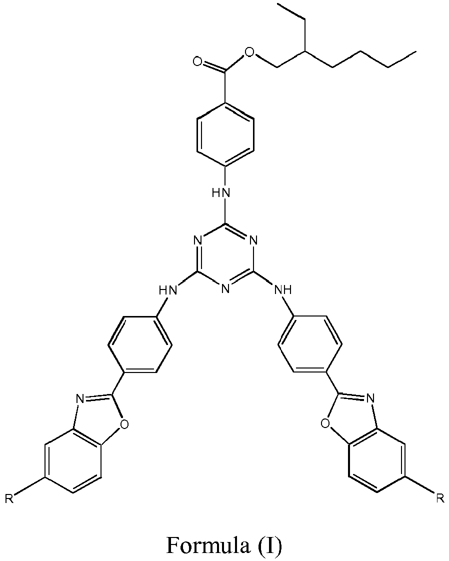 wherein the two R groups are independently branched C₅ alkyls.

The expression "branched C₅ alkyls" means branched alkyl chains containing five carbon atoms.

The compounds of formula (I) are useful as photostabilising agents against UV-visible radiation.

Generally, p-aminophenylbenzoxazole groups cause the compounds of formula (I) to absorb mainly UV-A radiation, i.e. in the zone of light wavelengths ranging between 320 and 400 nm, while p-aminobenzoic groups cause the compounds of formula (I) to absorb mainly UV-B radiation, i.e. in the zone of light wavelengths ranging between 290 and 320 nm.

The compounds of formula (I), bearing two p-aminophenylbenzoxazole substituents and a p-aminobenzoic group, therefore exhibit high absorption in the interval between 320 and 400 nm, and also cover part of the interval between 290 and 320 nm.

Preferred compounds of formula (I) are those wherein the R groups, which are the same or different, are selected from:
tert-pentyl (2-methylbutan-2-yl or 1,1-dimethylpropyl or tert-amyl) of formula CH₃CH₂C(CH₃)₂-;
neo-pentyl (2,2-dimethylpropyl) of formula (CH₃)₃CCH₂-;
iso-pentyl (3-methylbutyl) of formula (CH₃)₂CHCH₂CH₂-;
sec-pentyl (pentan-2-yl) of formula CH₃CH₂CH₂CH(CH₃)-;
3-pentyl (pentan-3-yl) of formula (CH₃CH₂)₂CH-;

Even more preferably, the compound of the invention is that of formula (I), wherein R is the tert-pentyl (2-methylbutan-2-yl or 1,1-dimethylpropyl) group of formula CH₃CH₂C(CH₃)₂-, or the compound of formula (II):

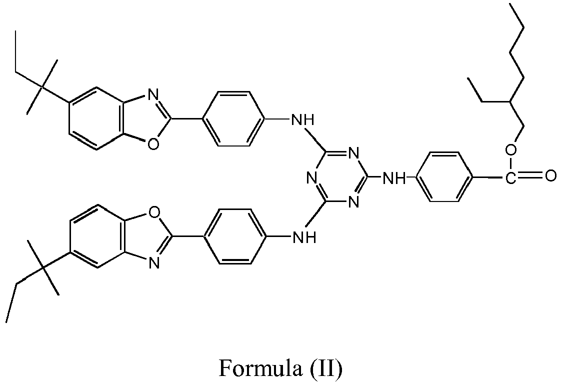

The triazine compounds of formula (I) can be obtained by reacting cyanuryl chloride or bromide with one equivalent of amine reagent of formula (III): 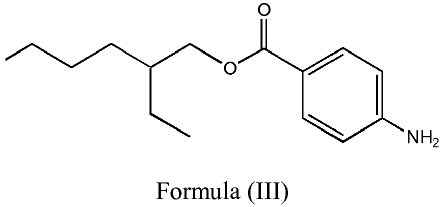 and two equivalents of a compound of formula (IV): 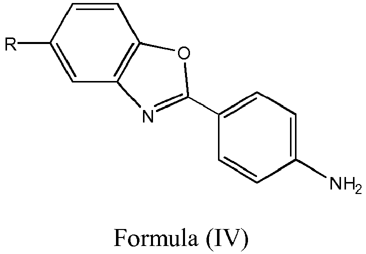 wherein R has the meanings previously described.

The compound of formula (III) is 2-ethylhexyl p-aminobenzoic acid ester.

An example of a compound of formula (IV) is 2-(4-aminophenyl)-5-(tert-amyl)benzoxazole.

The order in which the compounds of formulas (III) and (IV) are reacted with cyanuryl chloride or bromide can follow any intermediate sequence and stoichiometry. Cyanuryl chloride and bromide have three reactive halogen atoms able to react selectively with ammonia, primary amines and secondary amines at very different temperatures, thus making it possible to substitute each halogen atom with the desired amine with quantitative yields.

The subsequent synthesis for the preparation of triazine compounds from amino intermediates such as aminobenzoates and aminobenzamides is well known, and described, for example, in DE 3206398, US 4 617 390, US 4 724 137, US 5 233 040, US 5 252 323, US 5 332 568, IT 1255729, US 5 346 691, US 5 393 517, EP 832642, US 5 744 127, US 5 759 525, US 5 801 244, US 6 018 044, US 6 193 960, US 2002085981 and US 2005143577.

As the reaction produces acidity, neutralising bases are used in many cases, optionally in an aqueous medium, such as sodium hydroxide, sodium carbonate, sodium bicarbonate, calcium hydroxide, calcium carbonate, and tertiary amines such as triethylamine or pyridine.

The solvents wherein the compounds of the invention can be prepared need not be capable of dissolving the compounds. However, it is essential that they do not interact chemically with the compounds under the reaction conditions. In this respect they must be inert. Examples of solvents which can be used are saturated linear and branched hydrocarbons such as hexane, cyclohexane, methylcyclohexane, heptane, octane, isooctane, decane, petrols and dearomatised white spirits, aromatic hydrocarbons such as benzene, toluene, xylenes, ethylbenzene and petrols and white spirits also containing aromatic hydrocarbons, ketones such as acetone, methyl ethyl ketone, methyl isobutyl ketone and diisobutyl ketone, ethers such as tetrahydrofuran and dioxane, esters such as ethyl acetate and butyl acetate, and nitriles such as acetonitrile and benzonitrile.

Different solvents can also be used for the various consecutive synthesis steps, optionally isolating the intermediate compounds.

The operating temperatures range between 0°C and 200°C, preferably between 0 and 150°C. The pressures can range between 0 and 50 bars, preferably between 0 and 5 bars.

The compounds of the invention can be advantageously included in cosmetic formulations, either as the only sunscreen or in combination with other known sunscreens. These formulations are a further object of the invention.

Said formulations will preferably contain one or more conventional UV-A and UV-B sunscreens such as those listed in Annex VII to the European Cosmetics Directive (76/768/EEC) and Annex VI to European Regulation (EC) No. 1223/2009, as amended, in Household and Personal care Monographic Special Issue Skin Care - "The encyclopedia of allowed sunfilters in the world" by Giulio Pirotta and in ELECTRONIC CODE OF FEDERAL REGULATIONS (FDA) PART 352-SUNSCREEN DRUG PRODUCTS FOR OVER-THE-COUNTER HUMAN USE; Subpart B-Active Ingredients. Even more preferably, the formulations may contain, in addition to the derivatives of the invention, one or more sunscreens selected from 2-ethylhexyl p-methoxycinnamate, 2-hydroxy-4-methoxybenzophenone, 2-hydroxy-4-methoxybenzophenone-5-sulphonic acid, 2-phenylbenzimidazole-5-sulphonic acid and the sodium and potassium salts thereof, terephthalylidene dicamphor sulphonic acid, 2,2'-(1,4-phenylene)-bis(6-sulpho-1H-benzoimidazole-4-sulphonate) disodium salt, ethylhexyl salicylate, 2-hydroxy-benzoic acid 3,3,5-trimethylcyclohexyl ester, ethylhexyl dimethyl PABA, drometrizole trisiloxane, dimethicone-diethylbenzylmalonate, 3-(4'-methylbenzylidene)-d,1-camphor, diethylhexyl butamido triazone, ethylhexyl triazone, 4-(tert-butyl)-4'-methoxy-dibenzoylmethane, 2-cyano-3,3-diphenylacrylic acid 2-ethylhexyl ester, bis-ethylhexyloxyphenol-methoxyphenyl-triazine, methylene-bis-benzotriazolyl-tetramethylbutylphenol, benzoic acid 2-(4-diethylamino-2-hydroxybenzoyl)-hexyl ester, 2,4-bis-[4-[5-(1,1-dimethyl-propyl)benzoxazol-2-yl]phenylimino]-6-[(2-ethylhexyl)imi-no]-1,3,5-triazine, tris-biphenyltriazine, titanium dioxide and zinc oxide.

The compounds of the invention, alone or mixed with other sunscreens as just described, can also be formulated with light stabilisers (or SPF boosters) such as diethylhexyl-2,6-naphthalate, diethylhexyl syringylidene malonate, benzotriazolyl-dodecyl p-cresol, Polyester-8, butyloctyl salicylate and ethylhexyl methoxycrylene.

The compounds of the invention can also be used already in solution in the solvents allowed by European Regulation (EC) No. 1223/2009 on cosmetic products, as amended.

The invention will be now be described in greater detail in the following examples of cosmetic syntheses and preparations.

### Example 1: synthesis of 2,4-bischloro-6-(2-ethylhexyl-4-aminobenzoate)-1,3,5-triazine

37.0 g of sodium bicarbonate is added to a solution of 73.8 g of trichlorotriazine in 900 ml of acetone cooled to 0°C, and 102.2 g of 2-ethylhexyl-p-aminobenzoate is then slowly added, maintaining the temperature at 0°C by cooling. The mixture is then stirred for ½ hour, 240 ml of water is added and stirring continues for a further ½ hour; the precipitate formed is then filtered and washed several times with water, then with cold acetone, and dried under vacuum. 156.6 g of dichlorotriazine derivative is obtained in the form of a white solid with an MP of 245-248°C.

### Example 2: synthesis of 2,4-bis[5-tert-amylbenzoxazol-2-yl(4-phenyl)immo]-6-(2-ethylhexyl-4-aminobenzoate)-1,3,5-triazine

Compound of formula (I) with R = tert-amyl or compound of formula (II) of the present invention.

79.5 g of the bis-chlorocyanuryl intermediate of example I, 112.2 g of 2-(4-aminophenyl)-5-tert-amylbenzoxazole and 530 g of anhydrous xylene are stirred at 125°C for 3 hours in a nitrogen stream. The hydrochloric acid that develops is sent to a dilute solution of NaOH.

The mixture is cooled and neutralised with aqueous NaHCO₃, and the aqueous phase is separated. After drying, the mixture is clarified hot, and the product is isolated by complete hot removal of the solvent under vacuum. The resulting product is ground, obtaining 177 g of a whitish powder. The chemical structure is confirmed by NMR. The product has an HPLC assay value of 99.2% and an E¹₁ extinction = 1431 at 328 nm.

### Example 3 (comparative): synthesis of 2.4-bis[5-tert-butyl-benzoxazol-2-yl(4-phenyl)imino]-6-(2-ethylhexyl-4-aminobenzoate)-1,3,5-triazine

Compound of formula (I) with R = tert-butyl, described in example 24 of US 5 744 127.

Using a procedure similar to that of example 2, 27.8 g of the bis-chlorocyanuryl intermediate of example 1 and 37.3 g of 2-(4-aminophenyl)-5-tert-butylbenzoxazole are reacted, to obtain 49.2 g of end product as a whitish powder. The chemical structure is confirmed by NMR. The product has an HPLC assay value of 99.7% and an E¹₁ extinction = 1395 at 328 nm.

### Example 4 - Solubility test

The solubility of the sunscreens obtained in example 2 and (comparative) example 3 were evaluated in various emollients commonly used in suncare and daily skincare products: C12-15 alkyl benzoate, dibutyl adipate, caprylic/capric triglyceride and ethylhexyl salicylate.

**Table A**

| **Emollients (cosmetic solvents)** | **Compound of example 2 (w/w%)** | **Comparative compound of example 3 (w/w%)** |
|---|---|---|
| C12-15 Alkyl Benzoate | 15% | 5% |
| Dibutyl Adipate | 20% | 5% |
| Caprylic/Capric Triglyceride | 15% | <5% |
| Ethylhexyl Salicylate | 25% | 10% |

The results set out in Table A clearly show that the solubility of the compound of the present invention, with tert-amyl benzoxazole substituents, is greater than that of the similar compound described in example 24 of patent US5744127, with tert-butyl substituents. With the introduction of a branched C5 alkyl chain as described above, and the consequent increase in solubility in cosmetic oils, a sunscreen of formula (I) with greatly improved possibilities of use is therefore obtained. In particular, its high solubility produces protective sun oils with high SPF values, which cannot be achieved with the products of example 3 because of their lower solubility in said oils.

### Application examples

The compounds of example 2 of the present invention and the compound of (comparative) example 3 were tested for their ability to perform a photoprotective action.

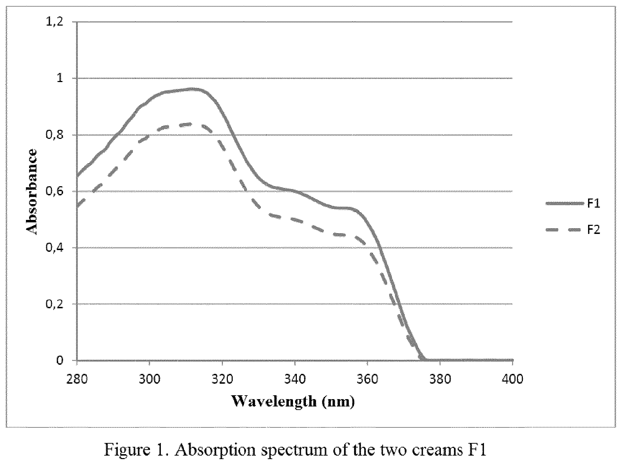

Said compounds were added to standard cosmetic formulas to evaluate the SPF (sun protection factor) value with a Labsphere UV-2000S instrument, in the UV region from 280 to 400 nm. For the measurement of the *in-vitro* SPF, the sun formulations were applied on a PMMA plate at a concentration of 2.0 mg/cm². Three different substrates were prepared for each formula tested, and 9 readings per substrate were conducted. The SPF data measured are set out in Tables 1, 2 and 3 relating to each application example. The instrument also produces the UV absorption spectra of the formulations shown in Figures 1, 2 and 3, relating to each application example.

### Example 5 - Application - Sun cream

**Table 1. Sun cream formulations F1 and F2 and the corresponding in-vitro SPF values.**

| **Phase** | **Ingredients** | **F1 w/w%** | **F2 w/w%** |
|---|---|---|---|
| I | Aqua | 70.50% | 70.50% |
| | Glycerin | 3.00% | 3.00% |
| | Abiol | 0.30% | 0.30% |
| II | Caprylic Capric/Triglyceride | 5.00% | 5.00% |
| | C12-15 Alkyl Benzoate | 4.00% | 4.00% |
| | Ethylhexyl Salicylate | 5.00% | 5.00% |
| | **Sunscreen of example 2** | **2.50%** | **/** |
| | **Sunscreen of comparative example 3** | **/** | **2.50%** |
| | HEB | 5.00% | 5.00% |
| | PEG-100 Stearate And Glyceryl Stearate | 3.00% | 3.00% |
| | Cetearyl Alcohol | 1.00% | 1.00% |
| | Behenyl Alcohol | 0.50% | 0.50% |
| III | Carbomer | 0.20% | 0.20% |
| IV | NaOH | q.s. | q.s. |
| Mean SPF | | **7.6** | **5.8** |

Preparation: Phase I and phase II were heated to 75-80°C under stirring. When phase II reached the temperature of 75°C, the carbomer was dispersed; phase II+III was then added to phase I, and the mixture was emulsified with the turbo emulsifier. The formulation was concluded with the addition of sodium hydroxide, and then brought to room temperature under stirring.

Table 1 and Figure 1 show that the two formulas in emulsion obtained with the two sunscreens have different absorption values and therefore different *in-vitro* SPF values, the performance of the sunscreen of example 2 being superior to that of the sunscreen of comparative example 3.

### Example 6 - Application - Sun oil

**Table 2. Anhydrous sun oil formulations and corresponding in-vitro SPF values.**

| **Phase** | **Ingredients** | **F3 w/w%** | **F4 w/w%** |
|---|---|---|---|
| I | Caprylic/Capric Triglyceride | 20.0% | 20.0% |
| | Dicapryl Carbonate | 20.0% | 20.0% |
| | Dibutyl Adipate | 20.0% | 20.0% |
| | C12-15 Alkyl Benzoate | 10.0% | 10.0% |
| | Ethylhexyl Salicylate | 5.0% | 5.0% |
| | **Sunscreen of example 2** | **5.0%** | **-** |
| | **Sunscreen of comparative example 3** | **-** | **5.0%** |
| | Uvasorb HEB | 4.0% | 4.0% |
| II | Ethanol | 16% | 16% |
| Mean SPF | | **13.9** | **12.2** |

Preparation: Phase I was heated to 75-80°C under stirring until a clear oil was obtained. The formulation was brought to room temperature under stirring, then added with ethanol.

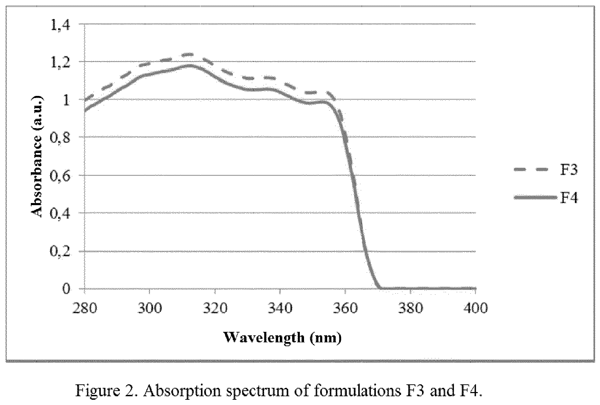

Table 2 and Figure 2, relating to oil formulations with medium sun protection, also exhibit differences in the absorption curve and *in-vitro* SPF favourable to formulation F3 containing the compound of example 2 of the invention. In particular, formulation F3 has a considerably higher SPF value and a higher UV absorption curve at all wavelengths.

### Example 7 - Application - Sun oil with high SPF

**Table 3. Anhydrous sun oil formulations and corresponding in-vitro SPF values.**

| **Phase** | **Ingredients** | **F4 w/w%** | **F5 w/w%** | **F6 w/w%** |
|---|---|---|---|---|
| I | Caprylic/Capric Triglyceride | 20.0% | 19.0% | 18.0% |
| | Dicapryl Carbonate | 20.0% | 18.0% | 16.0% |
| | Dibutyl Adipate | 20.0% | 19.0% | 18.0% |
| | C12-15 Alkyl Benzoate | 10.0% | 9.0% | 8.0% |
| | Ethylhexyl Salicylate | 5.0% | 5.0% | 5.0% |
| | **Sunscreen of example 2** | - | **10.0%** | **15.0%** |
| | **Sunscreen of comparative example 3** | **5.0%** | - | - |
| | Uvasorb HEB | 4.0% | 4.0% | 4.0% |
| II | Ethanol | 16% | 16% | 16% |
| Mean SPF | | **12.2** | **18.9** | **23.9** |

Preparation: Phase I was heated to 75-80°C under stirring until a clear oil was obtained. The formulation was brought to room temperature under stirring, and concluded with the addition of ethanol.

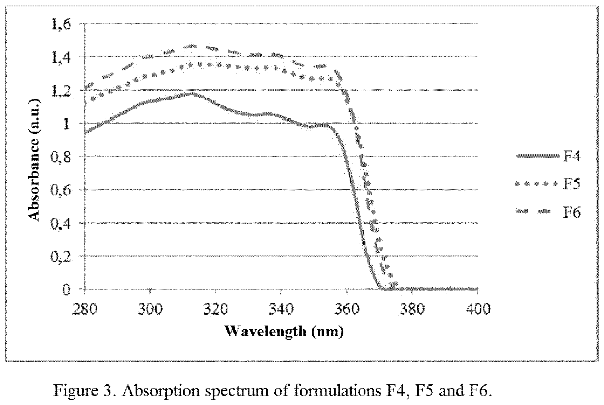

The higher solubility of the sunscreen of example 2 also allows the level of the sunscreen in the sun oil formulation to be increased, leading to a marked increase in both UVA and UVB sun protection in formulas F5 and F6 (Table 3 and Figure 3).

In the case of formulation F4 of the sunscreen of comparative example 3, it was impossible to increase the dose, because its low solubility limited its use. In fact, after only two weeks at 25°C after its production, formulation F4, unlike formulations F5 and F6, showed clear signs of precipitation of the sunscreen of comparative example 3. As in the above-mentioned case, the low solubility thereof leads to precipitation of the sunscreen in the formulation, prejudices its stability, and consequently reduces its SPF value.

## Claims

1. Compounds of formula (I): 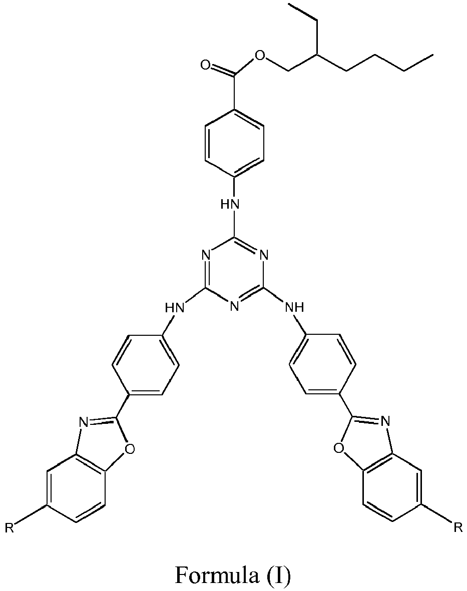 wherein the two R groups are independently branched C₅ alkyls.

2. Compounds according to claim 1 wherein each R is selected from the group consisting of:
tert-pentyl of formula CH₃CH₂C(CH₃)₂-;
neo-pentyl of formula (CH₃)₃CCH₂-;
iso-pentyl of formula (CH₃)₂CHCH₂CH₂-;
sec-pentyl of formula CH₃CH₂CH₂CH(CH₃)-;
3-pentyl of formula (CH₃CH₂)₂CH-;

3. Compound according to claims 1 and 2 of formula (II): 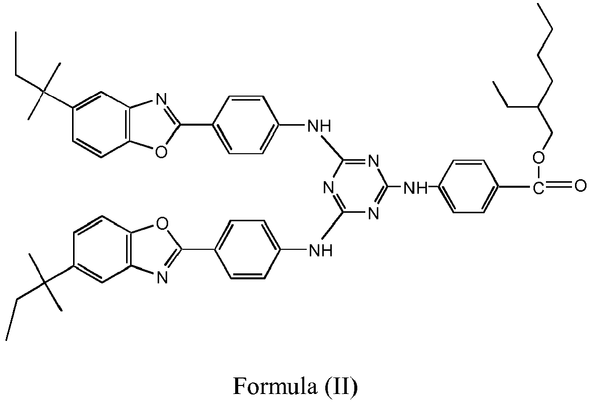 wherein R is tert-pentyl of formula CH₃CH₂C(CH₃)₂-.

4. The use of a compound according to claims 1 to 3 as a sunscreen against UV-A and UV-B radiation.

5. Cosmetic formulations containing at least one compound of claims 1 to 3.

6. Formulations according to claim 5 further containing one or more UV-A and UV-B sunscreens.

7. Formulations according to claim 6 containing at least one of the following sunscreens: 2-ethylhexyl p-methoxycinnamate, 2-hydroxy-4-methoxybenzophenone, 2-hydroxy-4-methoxybenzophenone-5 -sulphonic acid, 2-phenylbenzimidazole-5 - sulphonic acid and the sodium and potassium salts thereof, terephthalylidene dicamphor sulphonic acid, 2,2'-(1,4-phenylene)-bis(6-sulpho-1H-benzimidazole-4-sulphonate) disodium salt, ethylhexyl salicylate, 2-hydroxy-benzoic acid 3,3,5-trimethylcycloehexyl ester, ethylhexyldimethyl PABA, drometrizoletrisiloxane, dimethicone-diethylbenzalmalonate, 3-(4'-methylben-zylidene)-d,1-camphor, diethylhexylbutamidotriazone, ethylhexyltriazone, 4-(tert-butyl)-4'-methoxydibenzoylmethane, 2-cyano-3,3-diphenylacrylic acid 2-ethyl-hexyl ester, bis-ethylhexyloxyphenol-methoxyphenyl-triazine, methylene-bis-benzotriazolyl-tetramethylbutylphenol, benzoic acid 2-(4-diethylamino-2-hydroxybenzoyl)-hexyl ester, 2,4-bis-[4-[5-(1,1-dimethyl-propyl)benzoxazol-2-yl]phenylimino]-6-[(2-ethylhexyl)imino]-1,3,5-triazine, tris-biphenyltriazine, titanium dioxide and zinc oxide.

8. Formulations according to claims 5-7 further containing one or more light stabilisers.

9. Formulations according to claims 5-8 containing the light stabilisers diethylhexyl-2,6-naphthalate, diethylhexyl syringylidene malonate, benzotriazolyl-dodecyl p-cresol, Polyester-8, butyloctyl salicylate and ethylhexyl methoxycrylene.

10. Cosmetic formulations according to claims 5 to 9 in the form of creams or milky emulsions.

11. Formulations according to claims 5 to 9 in the form of oils.

12. Formulations according to claims 5 to 11 with an *in-vitro* SPF equal to or higher than 15.

13. A process for the preparation of compounds of formula (I) which comprises the reaction of cyanuryl chloride or bromide with one equivalent of an amino reagent of formula (III): 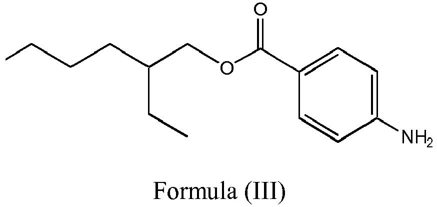 and two equivalents of a compound of formula (IV): 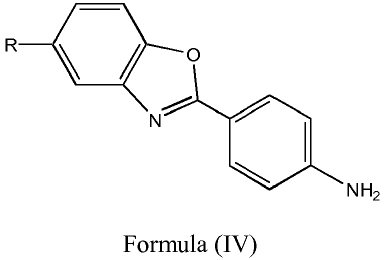 wherein R has the meanings described above.

## Patentansprüche

1. Verbindungen der Formel (I): 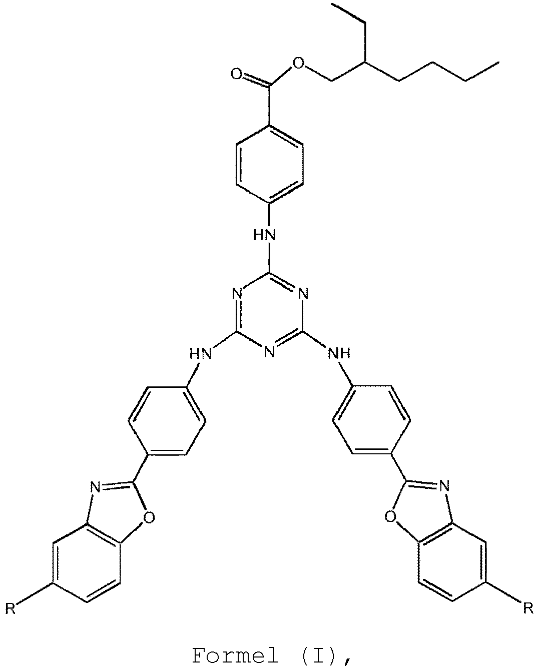 wobei die zwei R-Gruppen unabhängig voneinander verzweigte C₅-Alkyle sind.

2. Verbindungen nach Anspruch 1, wobei jedes R ausgewählt ist aus der Gruppe, bestehend aus:
tert-Pentyl der Formel CH₃CH₂C(CH₃)₂-;
Neopentyl der Formel (CH₃)₃CCH₂-;
Isopentyl der Formel (CH₃)₂CHCH₂CH₂-;
sec-Pentyl der Formel CH₃CH₂CH₂CH(CH₃)-;
3-Pentyl der Formel (CH₃CH₂)₂CH-;

3. Verbindung nach den Ansprüchen 1 und 2 der Formel (II): 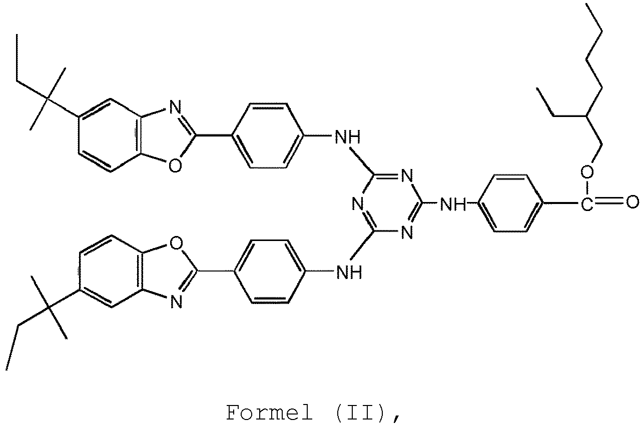 wobei R tert-Pentyl der Formel CH₃CH₂C(CH₃)₂- ist.

4. Verwendung einer Verbindung nach den Ansprüchen 1 bis 3 als Sonnenschutzmittel gegen UV-A- und UV-B-Strahlung.

5. Kosmetische Formulierungen, die mindestens eine Verbindung der Ansprüche 1 bis 3 enthalten.

6. Formulierungen nach Anspruch 5, die ferner ein oder mehrere UV-A- und UV-B-Sonnenschutzmittel enthalten.

7. Formulierungen nach Anspruch 6, die mindestens eines der folgenden Sonnenschutzmittel enthalten: 2-Ethylhexyl-p-methoxycinnamat, 2-Hydroxy-4-methoxybenzophenon, 2-Hydroxy-4-methoxybenzophenon-5-sulfonsäure, 2-Phenylbenzimidazol-5-sulfonsäure und die Natrium- und Kaliumsalze davon, Terephthalylidendicamphersulfonsäure, 2,2'-(1,4-Phenylen)-bis(6-sulfo-1H-benzimidazol-4-sulfonat)-dinatriumsalz, Ethylhexylsalicylat, 2-Hydroxybenzoesäure-3,3,5-trimethylcycloehexyles-ter, Ethylhexyldimethyl-PABA, Drometrizoltrisiloxan, Dimethicon-diethylbenzalmalonat, 3-(4'-Methylben-zyliden)-d,1-campher, Diethylhexylbutamidotriazon, Ethylhexyltriazon, 4-(tert-Butyl)-4'-methoxydibenzoylmethan, 2-Cyano-3,3-diphe-nylacrylsäure-2-ethylhexylester, Bisethylhexyloxyphenol-methoxyphenyl-triazin, Methylen-bis-benzotriazolyl-tetramethylbutylphenol, Benzoesäure-2-(4-dietylamino-2-hydroxybenzoyl)-hexylester, 2,4-Bis-[4-[5-(1,1-dimethylpropyl)benzoxazol-2-yl]phenylimino]-6-[(2-ethylhexyl)imino]-1,3,5-triazin, Trisbiphenyltriazin, Titandioxid und Zinkoxid.

8. Formulierungen nach den Ansprüchen 5 bis 7, die ferner einen oder mehrere Lichtstabilisator(en) enthalten.

9. Formulierungen nach den Ansprüchen 5 bis 8, die die Lichtstabilisatoren Diethylhexyl-2,6-naphthalat, Diethylhexylsyringylidenmalonat, Benzotriazolyl-dodecyl-p-kresol, Polyester-8, Butyloctylsalicylat und Ethylhexylmethoxycrylen enthalten.

10. Kosmetische Formulierungen nach den Ansprüchen 5 bis 9 in Form von Cremes oder milchigen Emulsionen.

11. Formulierungen nach den Ansprüchen 5 bis 9 in Form von Ölen.

12. Formulierungen nach den Ansprüchen 5 bis 11 mit einem *in-vitro-SPF* von gleich oder höher 15.

13. Verfahren zur Herstellung von Verbindungen der Formel (I), umfassend die Umsetzung von Cyanurylchlorid oder -bromid mit einem Äquivalent eines Aminoreagens der Formel (III): 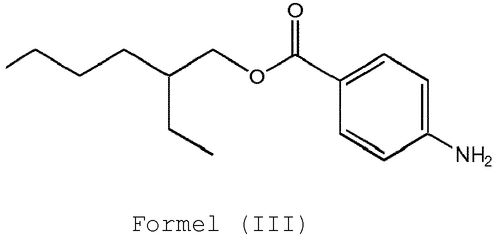 und zwei Äquivalenten einer Verbindung der Formel (IV): 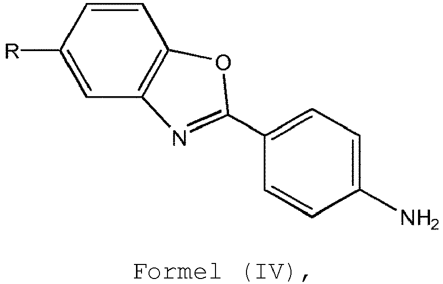 wobei R die oben beschriebenen Bedeutungen hat.

## Revendications

1. Composés répondant à la formule (I) : 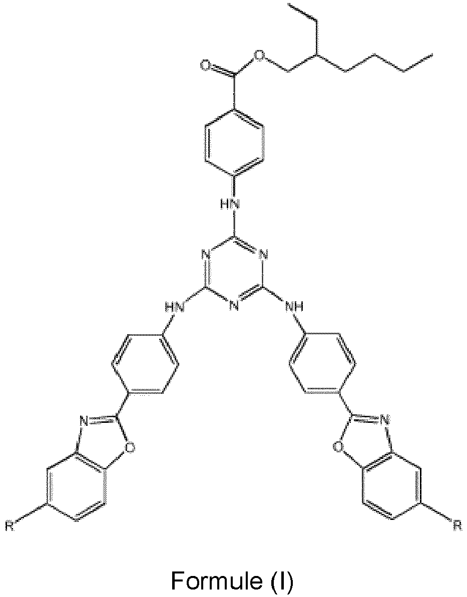 dans laquelle les deux groupes R représentent de manière indépendante des groupes alkyle en C₅ à chaîne ramifiée.

2. Composés selon la revendication 1, dans lesquels chaque R est choisi parmi le groupe constitué par :
un groupe tert-pentyle répondant à la formule CH₃CH₂C(CH₃)₂-;
un groupe néo-pentyle répondant à la formule (CH₃)₃CCH₂-;
un groupe iso-pentyle répondant à la formule (CH₃)₂CHCH₂CH₂-;
un groupe sec-pentyle répondant à la formule CH₃CH₂CH₂CH(CH₃)-;
un groupe 3-pentyle répondant à la formule (CH₃CH₂)₂CH-.

3. Composé selon les revendications 1 et 2, répondant à la formule (II) : 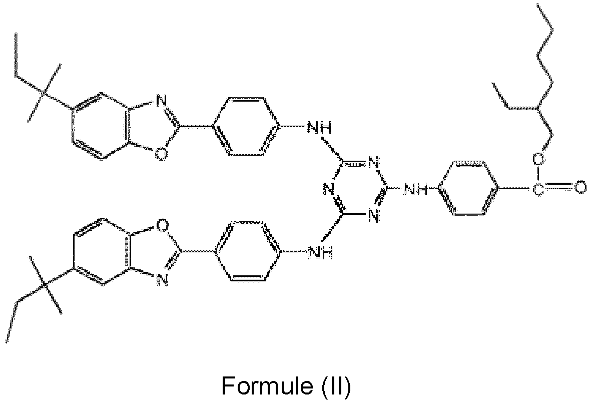 dans laquelle R représente un groupe tert-pentyle répondant à la formule CH₃CH₂C(CH₃)₂-.

4. Utilisation d'un composé selon les revendications 1 à 3 à titre d'écran solaire procurant une protection contre les rayonnements UV-A et UV-B.

5. Formulations cosmétiques qui contiennent au moins un composé selon les revendications 1 à 3.

6. Formulations selon la revendication 5, qui contiennent en outre un ou plusieurs écrans solaires procurant une protection contre les rayonnements UV-A et UV-B.

7. Formulations selon la revendication 6, qui contiennent au moins un des écrans solaires indiqués ci-après : le p-méthoxycinnamate de 2-éthylhexyle, la 2-hydroxy-4-méthoxybenzophénone, l'acide 2-hydroxy-4-méthoxybenzophénone-5-sulfonique, l'acide 2-phénylbenzimidazole-5-sulfonique, ainsi que ses sels de sodium et de potassium, l'acide téréphtalylidène dicamphre sulfonique, le sel disodique du 2,2'-(1,4-phénylène)-bis(6-sulfo-1H-benzimidazole-4-sulfonate), le salicylate d'éthylhexyle, l'ester 3,3,5-triméthylcyclohexylique de l'acide 2-hydroxy-benzoïque, l'éthylhexyl diméthyle PABA, le drométrizole trisiloxane, le diméthicone diéthylbenzyl malonate, le 3-(4'-méthyl-benzylidène)-d,l-camphre, la diéthylhexyl butamido triazone, l'éthylhexyl triazone, le 4-(tert-butyl)-4'-méthoxy-dibenzoylméthane, l'ester 2-éthyl-hexylique de l'acide 2-cyano-3,3-diphénylacrylique, la bis-éthylhexyloxyphénol-méthoxyphényl-triazine, le méthylène-bis-benzotriazolyl-tétraméthylbutylphénol, l'ester 2-(4-diéthylamino-2-hydroxybenzoyl)-hexylique de l'acide benzoïque, la 2,4-bis-[4-[5-(1,1-diméthyl-propyl)benzoxazol-2-yl]phénylimino]-6-[(2-éthylhexyl)imino]-1,3,5-triazine, la tris-biphényltriazine, l'oxyde de titane et l'oxyde de zinc.

8. Formulations selon les revendications 5 à 7, qui contiennent en outre un ou plusieurs agents photostabilisants.

9. Formulations selon les revendications 5 à 8, qui contiennent les agents photostabilisants que sont le diéthylhexyl-2,6-naphtalate, le malonate de diéthylhexyl syringylidène, le benzotriazolyl-dodécyl p-crésol, le Polyester-8, le salicylate de butyloctyle et l'éthylhexyl méthoxycrylène.

10. Formulations cosmétiques selon les revendications 5 à 9 sous la forme de crèmes ou d'émulsions laiteuses.

11. Formulations selon les revendications 5 à 9 sous la forme d'huiles.

12. Formulations selon les revendications 5 à 11, dont le facteur de protection solaire (SPF) *in vitro* est égal ou supérieur à 15.

13. Procédé pour la préparation de composés répondant à la formule (I), qui comprend la mise en réaction de chlorure ou de bromure de cyanuryle avec un équivalent d'un réactif amino répondant à la formule (III) : 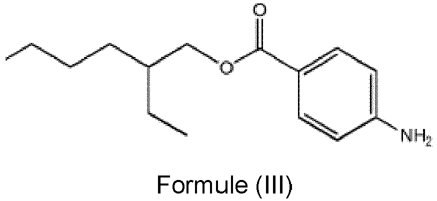 et avec deux équivalents d'un composé répondant à la formule (IV) : 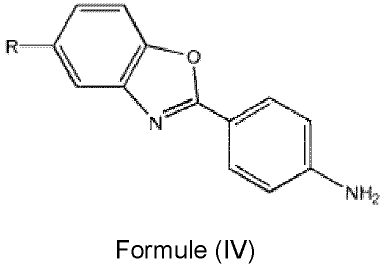 dans laquelle R
a les significations qui ont été décrites ci-dessus.
